# EUROPÄISCHE PATENTSCHRIFT

(11) **EP 1 277 831 B1**
(45) Veröffentlichungstag und Bekanntmachung des Hinweises auf die Patenterteilung: **22.11.2006**
(21) Anmeldenummer: 02090240.9
(22) Anmeldetag: 10.07.2002
(51) Int. Cl.: C12M 1/00, C12M 1/09, C12M 1/42

(54) **Verfahren und Vorrichtung zur Ernte mikrobieller Biomasse aus einem Kultivationssystem**
Method and apparatus for microbial biomass production from a cell culture system
Procédé et appareil de production de biomasse microbiènne à partir d'un système de culture cellulaire

(30) Priorität: 20.07.2001 DE 10136645
(43) Veröffentlichungstag der Anmeldung: 22.01.2003
(73) Patentinhaber: IGV Institut für Getreideverarbeitung GmbH, 14558 Bergholz-Rehbrücke (DE)
(72) Erfinder: Sandau, Ekkehard, 14558 Bergholz-Rehbrücke (DE); Pulz, Otto Dr. Dr. h.c., 14558 Bergholz-Rehbrücke (DE); Gutjahr, Jürgen, 14558 Bergholz-Rehbrücke (DE); Dörfer, Gerhard, 14469 Potsdam (DE); Franke, Horst, 14974 Mietgendorf (DE)
(74) Vertreter: Schubert, Klemens

(56) Entgegenhaltungen:
- WO-A-91/05849
- WO-A-98/51618
- SU-A- 869 605
- US-A- 4 280 886
- US-A- 4 294 697
- US-A- 6 000 551

## Beschreibung

Die Erfindung betrifft ein Verfahren zur Ernte von in einem offenen oder geschlossenen Kultivationssystem, vorzugsweise einem Bioreaktor, in wässriger Nährlösung kultivierter mikrobieller Biomasse. Sie bezieht sich vorzugsweise auf die Ernte von Mikroalgen. Weiterhin ist Gegenstand der Erfindung eine zur Durchführung des Verfahrens geeignete Vorrichtung.

Mikroorganismen, insbesondere Mikroalgen, kommt im Zusammenhang mit der Verwendung in Nahrungsmitteln bzw. Nahrungsergänzungsmitteln oder in Futtermitteln auf Grund der hohen Dichte ernährungsphysiologisch relevanter Inhaltsstoffe, aber auch im Hinblick auf die Verwendung in Medikamenten und

Kosmetika, eine zunehmende Bedeutung zu. Zur Bereitstellung größerer Mengen derartiger Mikroorganismen werden diese daher bereits seit einiger Zeit in speziellen Bioreaktoren kultiviert. Bei der industriellen Produktion der für die angegebenen Zwecke verwendbaren Biomasse bereitet jedoch die Ernte aus der zur Kultivierung in den Bioreaktoren verwendeten Nährlösung Probleme im Hinblick auf die ökonomische Effizienz der bislang hierbei eingesetzten Verfahren. Die Ernte erfolgt bisher beispielsweise mittels relativ teurer Separatoren unter Nutzung des Zentrifugalprinzips oder in einem mehrstufigen Sedimentations- und Trocknungsprozess. Unterstützend werden zur Koagulation bzw. Flokkulation der Biomasse in manchen Fällen chemische Flokkulationsmittel zugegeben, wobei dies jedoch den Nachteil mit sich bringt, dass die verwendeten Chemikalien zumindest teilweise in der Biomasse verbleiben.

Eine beispielhafte Lösung zur Kultivierung und Ernte von Mikroalgen wird durch die WO 91/05849 A1 offenbart. Die Schrift beschreibt ein Verfahren und eine Vorrichtung zur Produktion photosynthetisierender Mikroorganismen. Entsprechend der offenbarten Lehre werden die Mikroorganismen in an sich bekannter Weise in einem Photobioreaktor kultiviert. Die solchermaßen durch Photosynthese erzeugte Biomasse wird aus der Photosyntheseeinheit ausgetragen, flokkuliert und mittels einer mehrstufigen Sedimentationsanordnung aus der Suspension extrahiert. Die Flokkulation erfolgt durch Zugabe eines chemischen Flokkulationsmittels oder mittels eines vor den Sedimentationstanks angeordneten, von der Suspension in horizontaler Richtung durchflossenen Elektroflokkulators. Insbesondere bedingt durch die Mehrstufigkeit der zur Algenernte verwendeten Vorrichtung ist das Verfahren zeitintensiv und der Aufbau der Anlage vergleichsweise aufwendig und teuer.

Eine weitere mehrstufige Lösung ist aus der US 4,280,886 bekannt. In der Schrift werden ein Verfahren und eine Vorrichtung zur Aufbereitung von Abwasser beschrieben. Die Vorrichtung umfasst eine erste Stufe, in welcher in dem Abwasser enthaltene organische Substanzen durch Einbringung druckbeaufschlagten Wassers flokkuliert werden. Der Flokkulationsstufe schließen sich zwei Flotationsstufen an, wobei zunächst über eine Sprühdüse Luftblasen in das Abwasser mit der flokkulierten Biomasse eingetragen und die Biomasse schließlich in der letzten Stufe auf elektrolytischem Wege von der Flüssigkeit separiert wird. Bei der elektrolytischen Trennung von Abwasser und Biomasse setzt sich die Biomasse in der entsprechenden Flotationsstufe als Schaum an der Oberfläche ab und kann hier abgeskimmt werden. Abgesehen davon, dass die Vorrichtung nicht zum Zwecke der Ernte kultivierter Biomasse konzipiert ist, besitzt sie einen vergleichsweise komplizierten Aufbau, wobei ihre einzelnen Stufen in horizontaler Folge angeordnet sind.

Aus der US 5,951,875 ist eine ebenfalls mehrstufige Vorrichtung zur Extraktion von Beta-Karotin bekannt, bei der die Algen mit dem Beta-Karotin aus dem Wasser eines natürlichen Reservoirs (Süß- oder Salzwassersee) unter Einbeziehung einer Zentrifuge getrennt und aus dem hierbei entstehenden Konzentrat durch die Bildung von Gasblasen an die Oberfläche eines Behältnisses transportiert werden. Die Gasblasen werden mittels eines eingeleiteten Gases oder eines elektrischen Stromes durch die Suspension erzeugt, wobei dies manuell durch ein rotierendes Flügelrad unterstützt wird. Die beschriebene Vorrichtung ist zwar im Hinblick auf das gewünschte Resultat variabel auslegbar, aber verhältnismäßig aufwendig, zumal sie der Ernte von Biomasse aus natürlichen Vorkommen dient.

Zum Austragen von Biomasse aus einer Suspension ist das Prinzip der Separtion mittels Gasblasen (bubble separation) bereits bekannt. Entsprechende Anordnungen beziehungsweise Vorrichtungen sind beispielsweise aus der US 6,000,551 und der WO 98/51618 A1 bekannt. Gemäß dem in der erstgenannten US-Schrift beschriebenen Verfahren zur Trennung von Mikroalgen aus einer Suspension erfolgt die Flotation entweder mit Hilfe eines pneumatischen Gaseintrags in die Suspension oder durch die Erzeugung zur Flotation dienender Gasblasen mittels eines mechanischen Rotors. Die in der WO 98/51618 A1 beschriebene Lösung betrifft die Entfernung von Biomasse aus einer Flüssigkeit, vorzugsweise aus dem Wasser eines Aquariums. Gemäß dieser Lösung wird die mit Hilfe von Gasblasen erfolgende Flotation durch den Eintrag eines Luft-Ozon-Gemisches in die Flüssigkeit bewirkt. Durch das Luft-Ozon-Gemisch wird die Flüssigkeit derart verwirbelt, dass sich die Feststoffe in einer Schaumsäule in einem Behälteraußenbereich ansammeln, mit welcher sie über ein Steigrohr ausgetragen werden. Weder für die Flotation noch für die der Flotation gegebenenfalls vorausgehende Flokkulation kommen bei den in den beiden vorgenannten Schriften beschriebenen Lösungen elektrokinematische Mechanismen zum Einsatz.

Durch die SU 869 605 wird eine Vorrichtung offenbart, bei welcher die Trennung der Biomasse durch Elektroflokkulation und eine sich anschließende Elektroflotation erfolgt. Die Flokkulationszone und die Flotationszone sind in einem gemeinsamen Behälter beziehungsweise Tank einander horizontal folgend angeordnet. Beide Zonen sind durch eine vertikale, im Bereich des Behälterbodens unterbrochene Wand voneinander getrennt, so dass die Flokkulation und die Flotation als im Grunde getrennte beziehungsweise eigenständige Vorgänge nacheinander ablaufen.

Aufgabe der Erfindung ist es, ein Verfahren anzugeben, mittels welchem mikrobielle Biomasse aus einer in einem Kultivationssystem, vorzugsweise einem Bioreaktor, zur Kultivierung verwendeten Nährlösung in einfacher Weise effizient sowie in größeren Mengen geerntet werden kann. Die Aufgabe besteht weiterhin in der Schaffung einer zur Durchführung des Verfahrens geeigneten Vorrichtung, welche einen einfachen Aufbau besitzt und in bestehenden Anlagen (Bioreaktoren) mit geringem Aufwand nachrüstbar ist.

Die Erfindung wird durch ein Verfahren mit den Merkmalen des Hauptanspruchs gelöst. Die zur Durchführung des Verfahrens verwendbare Vorrichtung wird durch den ersten, unabhängigen vorrichtungsbezogenen Nebenanspruch charakterisiert. Vorteilhafte Aus- bzw. Weiterbildungen der Erfindung sind durch die jeweiligen Unteransprüche gegeben.

Nach dem erfindungsgemäßen Verfahren erfolgt die Ernte der mikrobiellen Biomasse im Grunde ausschließlich unter Nutzung elektrokinetischer Mechanismen. Sie erfolgt in einem kontinuierlichen Prozess mittels einer nach dem Durchflussprinzip arbeitenden Bypassanordnung zum Kultivationssystem - im Weiteren zumeist als Bioreaktor bezeichnet. Hierbei wird die Suspension mit der Biomasse zunächst als Volumenstrom vertikal durch ein als Flokkulationszone wirkendes Elektrodensystem geleitet. Die in dieser Zone unter Einwirkung einer an den Elektroden anliegenden Gleichspannung flokkulierende Biomasse wird dann in einer sich anschließenden Flotationszone mittels eines Gasbläschenstroms an die Oberfläche der Suspension getragen, wobei die Flotation durch bereits bei der Elektroflokkulation entstehende Gasbläschen unterstützt wird. In der Flotationszone wird die Biomasse in Form eines Schaums angereichert und kann schließlich abgeschöpft, abgenommen oder über eine Ablaufrinne abgeführt werden, während die aus der Suspension verbleibende Flüssigkeit in den Bioreaktor zurückgeführt wird. Als erfindungswesentlich ist es dabei anzusehen, dass der Gasbläschenstrom in der Flotationszone ebenfalls elektrolytisch mittels eines weiteren Elektrodensystems erzeugt wird. Die Ausnutzung elektrochemischer Vorgänge für die Flokkulation ist zwar als solches bekannt, jedoch geht die Erfindung einen Schritt weiter, indem sie elektrokinetische Mechanismen auch für die weitere Verfahrensführung nutzt und dadurch den Vorgang der Flokkulation in vorteilhafter Weise mit dem Vorgang des Austragens der flokkulierten Biomasse koppelt bzw. in diesen überleitet. Die Verfahrensführung gestaltet sich hierdurch besonders einfach, so dass sie, wie noch zu zeigen sein wird, mit einer Vorrichtung vergleichsweise einfachen Aufbaus zu realisieren ist. Bei der Flokkulation macht sich die Erfindung bekannte Aspekte der Zellbeschaffenheit der hier in Rede stehenden Biomasse und daraus ableitbare Möglichkeiten der Beeinflussung des Verfahrensregimes zunutze. So bestehen beispielsweise Zellwände von Mikroalgen aus einer festen fibrillären und einer amorphen Fraktion. Neben verschiedenen komplexen Polysacchariden als Hauptbestandteil der amorphen schleimigen Matrix und Fibrillen aus mikrokristalliner Zellulose enthalten Zellwände auch Proteine und Lipide. Diese Zellwandbestandteile enthalten Molekühle, die ionisationsfähige Gruppen, wie z. B. COOH, OH₂, PO₄, NH₂, SO₄, SH, besitzen können. Mikroalgenzellen bilden wie hydrophile Kolloide im wässerigen Medium zunächst eine Solvathülle mit fest an die Oberfläche der Zelle gebundenen orientierten Dipolen des Lösungsmittels. Darüber hinaus besitzen bzw. entwickeln die Zellen außer der Hydrathülle ein elektrisches Oberflächenpotential. Diese Oberflächenladung kann einmal durch Dissoziation der amphoteren Aminosäuren der Zellwandproteine in NH₃+ bzw. COO- entstehen. Ist das umgebende Flüssigkeitsmedium sauer, werden Protonen, also positive potentialbestimmende Ladungsträger an die Carboxylgruppe adsorbiert. In neutralem oder basischem Bereich werden sie umgekehrt durch Protonenabspaltung und Hydroxylgruppenanlagerung an die Aminogruppe negativ aufgeladen.

Kohlenhydrate ohne dissoziierbare Gruppen sind primär ungeladen und in Flüssigkeiten stark hydratisiert. Sie entwickeln in Abhängigkeit von der Dicke des Hydratmantels eine Oberflächenladung durch pH-abhängige lonenadsorption. Insgesamt ist die Oberflächenladung der Mikroalgenzelle pH-abhängig und im Bereich der optionalen Kultivation (pH 6-7) deutlich negativ. Experimentell ist im wässerigen Medium die Oberflächenladung der Algenzelle nicht zugänglich. Dafür steht das Zeta-Potential, ein Wert, der sich aus der elektrophoretischen Mobilität errechnen lässt, zur Verfügung. Das Zeta-Potential hängt von der Größe dieser Oberflächenladung und der die Zellen umgebenden Nährlösung ab. Es ist demnach ein Maß für die elektrische Ladung der Zelle in einer elektrisch geladenen Umgebung (Ionen der Nährlösung). Das Wirkprinzip der Elektroflokkulation ergibt sich daher aus dem elektrokinetischen Spannungsgefälle (Zeta-Potential) an der Phasengrenze fest/flüssig (Algenzelle/Suspension), welches sich in der Ausbildung einer elektrischen Doppelschicht äußert. Bringt man die Algensuspension in ein mit zwei Elektroden ausgestattetes Gefäß, wandern die Zellen bei Einwirkung von Gleichstrom je nach Vorzeichen ihres Zeta-Potentials in Richtung der jeweils entgegengesetzt geladenen Elektrode. Dort kommt es durch elektrochemische Vorgänge zur Ladungsneutralität. Der Kontakt mit der entgegengesetzten Elektrode wird in der Praxis durch Turbulenzen beim Durchfluss durch das mit Elektroden bestückte Gefäß unterstützt. Wenn die Zellen ihre Ladung abgegeben haben wirken die Coulombschen Abstoßungskräfte der stabilen Suspension nicht mehr, d. h. die Suspensionsstabilität zerbricht. Die Zellen werden in der Folge durch die van der Waalschen Kräfte angezogen, sie koagulieren bzw. flokkulieren. Durch die Flokkulation entstehen Zellaggregationen, welche im Sinne einer Ernte einfacher aus dem zur Kultivierung verwendeten System zu entfernen sind.

Wie bereits dargestellt, wird die Suspension vertikal durch die Flokkulationszone geführt. Im Zuge der durch die spannungsbeaufschlagten Elektroden hervorgerufenen Flokkulation und der gleichzeitig stattfindenden Elektrolyse bildet sich auch Gas, durch welches die Suspension in einer Aufwärtsbewegung in die sich anschließende Flotationszone einströmt. Bereits während der Flokkulation bilden sich auch Gasbläschen, welche die nachfolgende, aufgrund ihres senkrechten Aufbaus im Wesentlichen oberhalb der Flokkulationszone ablaufende Flotation einleiten und unterstützen.

Um zu verhindern, dass sich während der zur Flokkulation und zur Flotation genutzten elektrochemischen Vorgänge an den jeweils hierzu verwendeten Elektroden Beläge bilden oder diese gar zuwachsen, wird bei deren Bestromung, entsprechend einer vorteilhaften Verfahrensführung, zyklisch die Polarität gewechselt.

Im Hinblick auf die Beschaffenheit der Biomasse kommt deren Wassergehalt Bedeutung zu. Dabei wird mit Blick auf einen sich an die Ernte anschließenden Trocknungsprozess angestrebt, die Biomasse mit einem möglichst bereits geringen Wasseranteil zu ernten. Gemäß einer vorteilhaften Gestaltung des Verfahrens wird daher der Wassergehalt der geernteten Biomasse durch Variation des Füllstandes in der Flotationszone in diesem Sinne beeinflusst.

Die Flokkulationsfähigkeit der mikrobiellen Biomasse in der Suspension wird durch unterschiedliche Faktoren bestimmt. Insbesondere spielt dabei der pH-Wert der Suspension eine wichtige Rolle. Bei Versuchen ist festgestellt worden, dass der Koagulationsprozess innerhalb eines bestimmten pH-Bereichs (ca. 7 bis 7,2) besonders gut und effizient in Gang gesetzt wird. In Fortführung des Verfahrens ist jedoch zum Erhalt günstiger Resultate der pH-Wert möglichst zu kontrollieren, um zu vermeiden, dass er sich durch die Elektrolyse übermäßig und somit nachteilig erhöht. Gemäß einer Weiterbildung des erfindungsgemäßen Verfahrens wird daher eine pH-Wert-Messung in das Verfahrensregime einbezogen, um hieraus mittels einer Mess- und Steuerelektronik Stellgrößen für eine günstige Verfahrensführung ableiten zu können. Mit diesen Stellgrößen lässt sich der Prozess durch Einwirkung am Bioreaktor selbst oder an der Erntevorrichtung gezielt beeinflussen. So kann beispielsweise der pH-Wert durch eine CO₂-Dosierung beeinflusst werden, wobei die Dosierung vorzugsweise am Bioreaktor selbst erfolgt, weil eine entsprechende Maßnahme an der zur eigentlichen Ernte verwendeten Vorrichtung deren Aufbau wieder verkomplizieren würde. Es wurden aber auch Einflüsse der Stromstärke durch die Elektroden festgestellt, so dass auch eine Einflussnahme auf den zeitlichen Verlauf der Stromstärke in Abhängigkeit des jeweils festgestellten pH-Werts zielführend sein kann. Auch zwischen der Leitfähigkeit und dem Zeta-Potential bestehen Zusammenhänge, und zwar derart, dass eine Erhöhung der Leitfähigkeit mit einer erwünschten Reduzierung des Zeta-Potentials der Zellen einhergeht. Folglich können in vergleichbarer Weise auch aus den Ergebnissen einer Messung der Leitfähigkeit, beispielsweise im Zuflussbereich zur Flokkulationszone, Stellgrößen für die Verfahrensführung gewonnen werden. Die Bestimmung der Leitfähigkeit kann somit alternativ zur pH-Wert-Messung erfolgen oder zu ihr hinzutreten.

In Weiterbildung des Verfahrens ist außerdem eine Messung der optischen Dichte der Suspension im Bereich des Ablaufs der verwendeten und nachfolgend erläuterten Vorrichtung, also nachgeordnet zu den Maßnahmen für die Ernte der Biomasse, vorgesehen. Aus dem Ergebnis dieser Messung lassen sich Rückschlüsse auf die Effizienz des Ernteverfahrens ziehen, da sie ein Maß für die Menge der in der Suspension verbliebenen Biomasse liefert.

Die zur Durchführung des Verfahrens geeignete Vorrichtung ist als Bypassanordnung mit einem Zulauf vom und einem Ablauf zum Bioreaktor ausgebildet. Hierdurch kann sie vorteilhafterweise einfach in bestehende Systeme zur Kultivierung von Biomasse eingefügt werden und stellt insoweit auch eine preisgünstige Lösung dar. Die Vorrichtung umfasst in einem gemeinsamen Gehäuse eine Flokkulationszone, eine vertikal oberhalb der Flokkulationszone angeordnete Flotationszone sowie eine sich an die Flotationszone in vertikaler Richtung anschließende Anreicherungszone. Bei der Flokkulationszone handelt es sich um einen mit dem Zulauf unmittelbar in Verbindung stehenden und von der Suspension durchflossenen Abschnitt mit einem Elektrodensystem von senkrecht verlaufenden, horizontal benachbart angeordneten Elektroden. Dieser Abschnitt ragt senkrecht in den nächsten sich in seiner horizontalen Erstreckung gegenüber der Flokkulationszone erweiternden und die Flotationszone ausbildenden Abschnitt hinein. Die Flotationszone ist aus strömungstechnischer Sicht mit dem Ablauf verbunden. In ihr ist mindestens eine Gitterelektrode waagerecht eingeordnet. Vorzugsweise handelt es sich aber auch hier um ein Elektrodensystem aus mehreren jeweils waagerecht und parallel zueinander angeordneten Gitterelektroden. In dem Bereich oberhalb der Gitterelektroden bildet sich im Abstand zu ihnen beim Betrieb der Vorrichtung an der Oberfläche der Flotationszone die Anreicherungszone aus.

Gemäß einer vorteilhaften Ausgestaltung der Erfindung sind die Stromkreise für das Elektrodensystem der Flokkulationszone und die Gitterelektroden in der Flotationszone voneinander unabhängig ausgeführt und werden von unabhängigen Stromquellen gespeist. Entsprechend einer praktikablen Ausbildung der Vorrichtung besteht das Elektrodensystem der Flokkulationszone aus einem eine äußere Elektrode ausbildenden metallischen Rohr und einer zweiten konzentrisch darin eingeordneten inneren Elektrode, welche als metallischer Rundstab oder ebenfalls als metallisches, jedoch an beiden Enden verschlossenes Rohr ausgebildet ist. Dabei wird die über den Zulauf zugeführte Suspension zur Flokkulation der Biomasse zwischen der äußeren und der inneren Elektrode hindurchgeleitet. Bei einer anderen, vorteilhafteren Ausführungsform der Vorrichtung wird das Elektrodensystem der Flokkulationszone von drei einander konzentrisch umgebenden Elektroden gebildet. Es besteht aus einer äußeren rohrförmigen Elektrode, in welche eine zweite, ebenfalls als Rohr ausgebildete Elektrode eingeordnet ist, sowie der dritten konzentrisch zu der zweiten Rohrelektrode angeordneten inneren Elektrode, wobei letztere wiederum als Rundstab oder ebenfalls verschlossene Rohrelektrode ausgebildet ist. Bei dieser Ausführungsform werden die äußere Rohrelektrode und die innere Elektrode an den gleichen Pol der Stromversorgung angeschlossen, während die dazwischen eingeordnete Rohrelektrode gegenpolig geschaltet wird. Durch diese Ausbildung wird die Vorrichtung sehr kompakt, da die Suspension in der Flokkulationszone zunächst zwischen den beiden Rohrelektroden und dann zwischen der inneren der beiden Elektroden sowie der in diese wiederum eingeordneten Innenelektrode vertikal geführt wird. Hierdurch verkürzt sich bei gleicher für den Flokkulationsprozess zur Verfügung stehender Elektrodenoberfläche die vertikale Erstreckung der Flokkulationszone etwa um die Hälfte. Der Zulauf mündet dabei waagerecht kurz unterhalb der Flotationszone in die Flokkulationszone ein. In vorteilhafter Weise können gemäß dieser Ausgestaltung sowohl Zulauf als auch Ablauf waagerecht auf etwa gleicher Höhe durch eine Grundplatte geführt werden, auf welcher der die Flotationszone mit den Gitterelektroden aufnehmende Vorrichtungsabschnitt montiert ist.

Die Vorrichtung ist dadurch vorteilhaft weitergebildet, dass die rohrförmige Flokkulationszone an dem Ende, an welchem sie in den die Gitterelektroden aufnehmenden Abschnitt hineinragt, von einer sich radial zur Gehäusewandung erstreckenden Scheibe umgeben ist. Die beiden großen Oberflächen dieser Scheibe laufen dabei aufeinander zu, wobei zumindest die Unterseite gegen die Waagerechte geneigt ist. Die Scheibe verhindert, dass die aus der Flokkulationszone in den Bereich mit den Gitterelektroden und der sich anschließenden eigentlichen Flotationszone einströmende Suspension sofort wieder über den am Boden dieses Bereiches vorgesehenen Ablauf abfließt. Nur so kann sichergestellt werden, dass die flokkulierte Biomasse von den an den Gitterelektroden vermehrt gebildeten Gasbläschen erfasst und durch diesen Gaslift zur Oberfläche der Flotationszone transportiert wird. Die vom Zentrum zum Rand nach oben gerichtete Neigung der Unterseite der ausgangs der Flokkulationszone vorgesehenen Scheibe ermöglicht es, Gasbläschen, welche sich in diesem Bereich ebenfalls bilden, nach oben zu entweichen und den Gaslift zu unterstützen. Dabei wird zudem verhindert, dass diese Gasblasen sich übermäßig vergrößern und hierdurch unerwünschte Turbulenzen in der Erntevorrichtung hervorrufen.

Die Entnahme der sich an der Oberfläche der Flotationszone in Form von Schaum anreichernde Biomasse erfolgt gemäß einer besonders vorteilhaften Ausgestaltung der Vorrichtung mittels einer an der Oberkante des Gefäßes, also der Flotationszone, angeordneten sowie leicht nach unten geneigt verlaufenden Ablaufrinne. Über diese Ablaufrinne gleitet der Schaum selbsttätig langsam in ein Auffanggefäß ab. Dabei wird der Füllstand in der Flotationszone vorteilhafterweise so bemessen, dass sich die Schaumschicht etwa 2 bis 5 cm unterhalb der Kante der Ablaufrinne nach oben erstreckend ausbildet. Durch Querrinnen am Boden der Ablaufrinne, über welche im Schaum verbliebene Flüssigkeit zur Seite abfließt, kann der gewünschte Erhalt eines möglichst trockenen Schaums zusätzlich begünstigt werden.

Zur verfahrensgünstigen Steuerung der Abläufe in der Erntevorrichtung sind an unterschiedlichen Stellen Sensoren vorgesehen und die von ihnen erfassten Größen werden einer mit der Vorrichtung verbundenen Mess- und Steuereinrichtung zugeführt. Neben der Messung des pH-Werts und/oder der Leitfähigkeit können dabei auch Mittel zur Erfassung der Füllstandshöhe in der Flotationszone vorgesehen sein, so dass die Füllstandshöhe im Ergebnis durchgeführter Messungen im Hinblick auf eine günstige Schaumdicke bzw. -höhe beeinflusst werden kann. Die Mittel zur Füllstandsmessung sind somit Bestandteil eines Regelungskreises, durch welchen insbesondere die Zufuhr von Suspension zur Erntevorrichtung und damit deren Volumenstrom anhand in der Mess- und

Steuereinrichtung abgeleiteter Stellgrößen (beispielsweise durch Regelung einer Pumpenleistung oder mittels zusätzlicher steuerbarer Ventile) beeinflusst wird. Die Zufuhr der Suspension erfolgt nämlich entsprechend einer Möglichkeit mittels einer Pumpe, durch weiche sie aus dem Sammei- und Ausgleichsbehälter des Bioreaktors zur Erntevorrichtung gefördert wird. Es ist jedoch auch möglich, auf der Druckseite einer zwischen dem Sammel- und Ausgleichsbehälter und der Photosyntheseeinheit eines als Photobioreaktor ausgebildeten Bioreaktors ohnehin vorhandenen Pumpe einen Abzweig vorzusehen und die Suspension über diesen zur Erntevorrichtung abzuleiten. Dies setzt allerdings voraus, dass die Durchflussmenge in diesem Abzweig unabhängig von dem im Reaktorkreislauf benötigten und mit der Pumpe erzeugten Durchsatz an Suspension regelbar ist. Jedoch sei an dieser Stelle betont, dass sich der Einsatz der Vorrichtung nicht auf die Verwendung zusammen mit Photobioreaktoren beschränkt. Sie stellt vielmehr eine kostengünstige, variabel - also auch zusammen mit anderen Kultivationssystemen bzw. Bioreaktoren - einsetzbare Lösung dar. Die Vorrichtung hat einen einfachen sowie robusten Aufbau. Wegen ihrer Kompaktheit hat sie einen geringen Platzbedarf und ein niedriges Gewicht. Zudem ist die Vorrichtung schnell zerlegbar und auch gut zu reinigen. Durch das Fehlen rotierender Teile wird im Betrieb keine Lärmbelästigung verursacht. Außerdem hat die Vorrichtung im Vergleich zu anderen bekannten Lösungen einen geringen Energiebedarf, so dass sich niedrige Betriebskosten ergeben.

Die Erfindung soll nachfolgend anhand eines Ausführungsbeispieles näher erläutert werden. In den zugehörigen Zeichnungen zeigen:
- Fig. 1:: Eine Prinzipdarstellung der erfindungsgemäßen Vorrichtung mit ihren wesentlichen Elementen teilweise geschnitten;
- Fig. 2:: Einige Elemente der Vorrichtung gemäß Fig. 1 in einer detaillierteren, ebenfalls teilweise geschnittenen Darstellung;
- Fig. 3:: Die erfindungsgemäße Vorrichtung in der Bypassanordnung zu einem als Photobioreaktor ausgebildeten Bioreaktor.

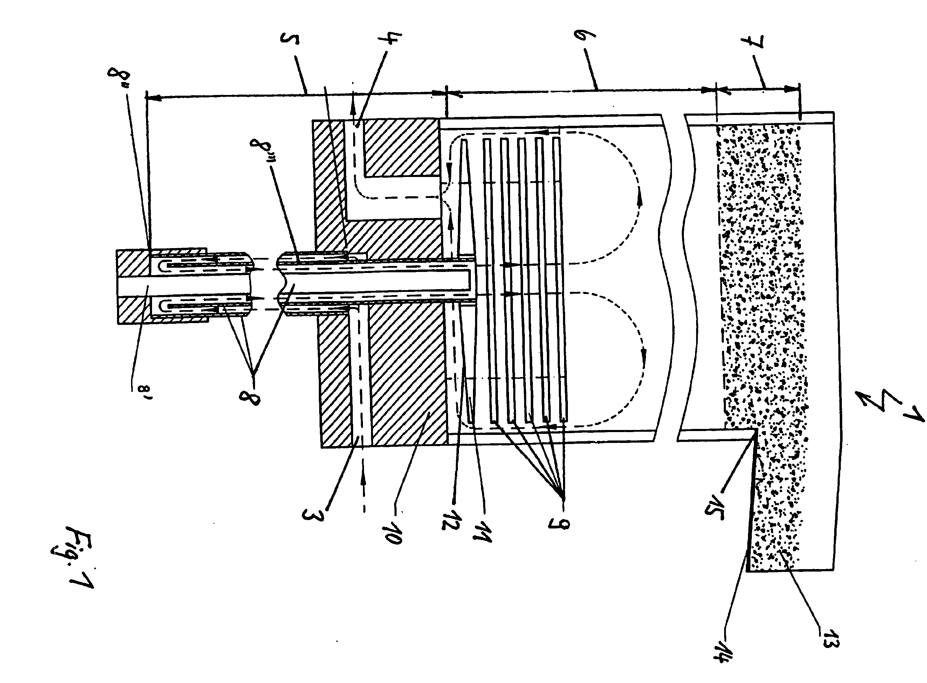

Die Fig. 1 zeigt die erfindungsgemäße Vorrichtung 1 mit ihren wesentlichen Elementen in teilweise geschnittener Darstellung, wobei die Darstellung in vertikaler Richtung teilweise unterbrochen wurde, so dass sie die tatsächlichen geometrischen Verhältnisse nicht exakt maßstabsgetreu wiedergibt. Die Vorrichtung 1 verfügt über einen Zu- und einen Ablauf 3, 4 für die Suspension, aus welcher die Biomasse geerntet werden soll. Sie umfasst eine Flokkulationszone 5, die sich vertikal daran anschließende Flotationszone 6 und einen Bereich an der Oberseite der Flotationszone 6, in dem sich beim Betrieb der Vorrichtung die Anreicherungszone 7 ausbildet. Die Flokkulationszone 5 wird durch ein drei Elektroden 8', 8", 8''' umfassendes Elektrodensystem 8 ausgebildet. Sie besitzt eine annähernd zylindrische Form, also einen etwa kreisrunden Querschnitt. Dabei wird eine innere Elektrode 8' mit zylindrischer Oberfläche (Stabelement oder beidseitig verschlossenes Rohr) von einer zweiten (Zwischen-)Elektrode 8"', mit ebenfalls zylindrischer Form, konzentrisch umschlossen. Beide Elektroden werden schließlich von einer dritten äußeren Rohrelektrode 8" umfasst. Durch die Zwischenräume der Elektroden 8', 8", 8''' wird die einströmende Suspension mit der Biomasse geführt. Sie wird der Vorrichtung 1 über den waagerecht verlaufenden Zulauf 3 zugeführt. Die Elektroden 8', 8", 8''' sind über hier nicht dargestellte Kabel in der Weise mit Spannung beaufschlagt, dass die äußere und die innere Elektrode 8', 8" jeweils die gleiche Polarität aufweisen, während die dazwischen eingeordnete Elektrode 8''' gegenpolig beschaltet ist. Beim Durchlauf der Suspension durch die Elektrodenzwischenräume kommt es zur Flokkulation.

Die entstehenden Algenaggregationen werden mit der Suspension in die sich anschließende Flotationszone 6 gespült, wobei dieser Vorgang durch sich bei der Flokkulation bildendes Gas unterstützt wird, dessen Bläschen im Grunde bereits auch die Flotation einleiten. In der Flotationszone 6 ist im Beispiel eine Mehrzahl von Gitterelektroden 9 angeordnet. Die Gitterelektroden 9 haben eine waagerechte Anordnung und sind, wie zu erkennen, zueinander parallel ausgerichtet. Von jeweils einer zur nächsten Gitterelektrode 9 wechselt im Betrieb der Vorrichtung die Polarität. Hierdurch kommt es im Zuge einer verstärkten Elektrolyse zur Bildung einer Vielzahl von Gasbläschen. An diese Gasbläschen lagern sich die Zellgruppen der Biomasse an und werden von Ihnen in einem Gaslift zur Oberfläche der Flotationszone 6 befördert. Als besonders günstig erweist sich dabei die gitterartige Ausbildung der Elektroden 9. Hierdurch kann sich ein über den gesamten Querschnitt der Flotationszone 6 verteilender Gasstrom ausbilden, dessen Bläschen die Zellflocken zur Oberfläche (am vertikal oberen Ende der Flotationszone 6) führen. Dabei können der Gasstrom und die flokkulierte Biomasse die siebartigen Elektroden 9 ganzflächig, nahezu ungehindert passieren. Die Flotationszone 6 ist strömungstechnisch mit dem Ablauf 4 verbunden, über welchen die Suspension nach der Ernte der Biomasse in den Bioreaktor 2 zurückgeführt wird. Damit die von der Flokkulationszone 5 her eintretende Suspension nicht sofort in diesen Ablauf 4 fließt, ist die Flokkulationszone 5 im Bereich ihres Hineinragens in die Flotationszone 6 von einer Scheibe 11 umgeben. Diese bildet quasi einen strömungstechnischen Schatten für die auf der Oberseite der Scheibe 11 einströmende Suspension. Die Abstände von den Gitterelektroden 9 zu den seitlichen Außenwänden bzw. der entsprechende Abstand der Scheibe 11 sind so gewählt, dass in diesen Bereichen keine höheren Strömungsgeschwindigkeiten entstehen, welche verstärkt Biomasseteilchen in Richtung des Auslaufs 4 mit sich reißen würden.

In besonders vorteilhafter Weise ist zumindest die Unterseite 12 der Scheibe 11 gegenüber der Horizontalen so geneigt, dass sich die Höhe der Scheibe 11, ausgehend vom Zentrum, zu den Rändern verringert. Dadurch können an der Unterseite 12 der Scheibe 11 ebenfalls entstehende Gasbläschen an deren Außenfläche entlanggleiten und schließlich im Bereich der Ränder nach oben entweichen. Auf diese Weise wird verhindert, dass es hier zur Bildung größerer Gasblasen kommt, welche zu unerwünschten Turbulenzen führen könnten. Die vom Gaslift zur Oberfläche der Flotationszone 6 geförderte Biomasse reichert sich dort in Form eines Schaums 13 an. Über eine Ablaufrinne 14 kann dieser Schaum langsam gleitend in ein (nicht dargestelltes) Auffanggefäß geführt werden oder in anderer Weise kontinuierlich abgeführt werden. Zum Erhalt einer möglichst trockenen Biomasse ist für die Schaumschicht 13 eine bestimmte Mächtigkeit (Schaumdicke bzw. -höhe) anzustreben. Vorteilhafterweise sollte dabei der Füllstand der Suspension in der Flotationszone 6 so gewählt werden, dass die Schaumschicht 13 ca. 2 bis 5 cm unterhalb der Kante 15 des Übergangs in die Ablaufrinne 14 beginnt. Dadurch kann im Schaum noch enthaltenes Wasser vor dem Abgleiten aus diesem austreten und in die Flotationszone 6 zurücklaufen.

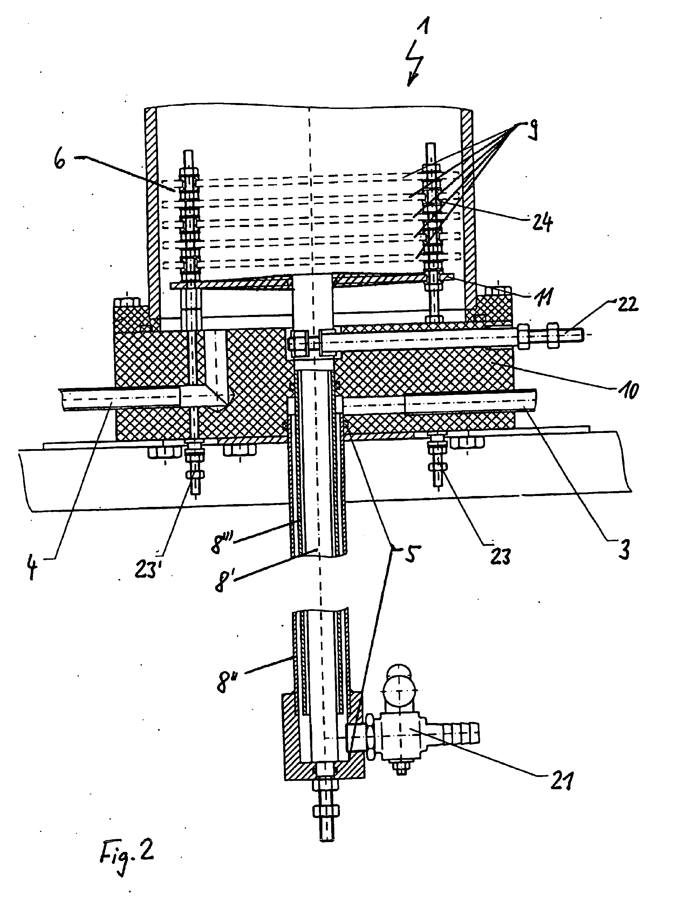

Die Fig. 2 zeigt einige wesentliche Elemente der Vorrichtung 1 nach Fig. 1 nochmals in einer etwas detaillierteren Darstellung. Insbesondere wird hier das Elektrodensystem 9 in der Flotationszone 6 besser verdeutlicht. Die Flotationszone 6 mit den zugehörigen Einbauten ist, wie zu erkennen, auf einer Grundplatte 10 montiert. Durch diese Grundplatte 10 sind in waagerechter Richtung der Zu- und der Ablauf 3, 4 für die Suspension geführt. In vertikaler Richtung wird die Platte 10 vom Elektrodensystem 8 der Flokkulationszone 5 durchragt. Die Bestromung der Gitterelektroden 9 erfolget über mit elektrischen Anschlüssen versehene, die Grundplatte 10 ebenfalls vertikal durchragende Stehbolzen 23, 23'. Zur Bestromung des Elektrodensystems 8 in der Flokkulationszone 5 sind waagerecht verlaufende Durchführungen 22 vorgesehen. In den Stromkreislauf der Gitterelektroden 9 ist die bereits erwähnte Scheibe 11 mit einbezogen. Hierdurch ist es grundsätzlich möglich, die Vorrichtung mit nur einer Gitterelektrode 9 auszubilden und einen Pol des zu ihrer Bestromung dienenden Stromkreises an diese Elektrode 9 sowie den anderen an die Scheibe 11 anzuschließen. Im Hinblick auf die Ausbildung eines besonders kräftigen Gasstroms sind jedoch mehrere Elektroden 9 von Vorteil, wobei die Scheibe 11 jeweils die gleiche Polarität wie die zweite über ihr angeordnete Gitterelektrode 9 hat. Das Elektrodensystem 8 der Flokkulationszone 5 wird beim Betrieb der Vorrichtung 1 ebenso wie die Gitterelektroden 9 mit einem hinsichtlich seiner Polarität zyklisch wechselnden Gleichstrom versorgt. Der zyklische Wechsel der Strompolarität erfolgt zur Vermeidung vorzeitiger Elektrodenalterung und von Ablagerungen an den Elektroden 8, 8', 8", 9. Insbesondere im Hinblick auf den freien Durchgang durch die Gitterelektroden 9 wird somit verhindert, dass diese mit sich ablagernder Biomasse zuwachsen. Vorzugsweise werden die Elektrodensysteme 8, 9 der Flokkulationszone 5 einerseits und der Flotationszone 6 andererseits über unabhängige Stromkreise versorgt. Dadurch können für beide Bereiche unabhängig voneinander die jeweils günstigsten Stromverhältnisse eingestellt werden. Die elektrischen Versorgung der Gitterelektroden 9 erfolgt, wie bereits erwähnt, durch beidseits vorhandene Stehbolzen 23, 23', welche an der Grundplatte 10 montiert sind und das gesamte Elektrodensystem 9 tragen, wobei die Gitterelektroden 9 gegeneinander durch Isolatoren 24 getrennt sind. Die in der Fig. 2 dargestellte Detailansicht ist gegenüber der Darstellung in der Fig. 1 um einen Ablasshahn 21 ergänzt, über welchen in der Vorrichtung 1 verbliebene Suspension bei deren Reinigung abgelassen werden kann.

Eine in der Erprobung erfolgreich betriebene Vorrichtung 1 hat eine Länge der Flokkulationszone 5 von ca. 1 m und, bezogen auf die äußere Rohrelektrode 8", einen Innendurchmesser von ca. 15 mm, während der Außendurchmesser der anderen Rohrelektrode 8"' (Zwischenelektrode) ca. 12 mm beträgt, so dass der Zwischenraum für die hindurchströmende Suspension ca. 1,5 mm beträgt. Bei Leistungsaufnahmen aller Elektroden 8', 8", 8"', 9 von in der Summe ca. 25 bis 40 W wurden bei einem Durchfluss von ca. 1 Liter pro Minute Abscheideraten zwischen ca. 60 und 85% erzielt.

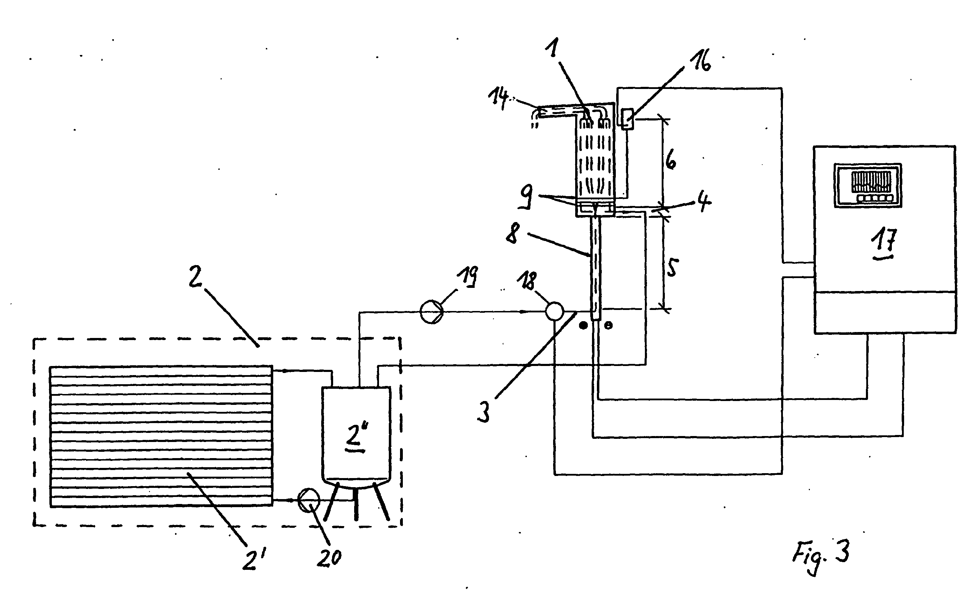

Durch die Fig. 3 wird die erfindungsgemäße Vorrichtung 1 in ihrer Anordnung als Bypasssystem zu einem Bioreaktor 2 dargestellt. Es handelt sich hierbei um einen Photobioreaktor. Selbstverständlich sind das Verfahren und die Vorrichtung 1 auch im Zusammenspiel mit anderen Kultivationssystemen bzw. Bioreaktoren einsetzbar. Durch die Bypassanordnung ist dabei eine besonders günstige Nachrüstbarkeit der Vorrichtung 1 für den Einsatz mit bereits vorhandenen Bioreaktoren gegeben. Gegenüber den Fig. 1 und 2 haben der Zu- und der Ablauf 3, 4 der Vorrichtung 1, ohne Veränderung des grundsätzlichen Aufbaus, lediglich eine etwas andere Anordnung. Der Zufluss der Suspension erfolgt hier vertikal von unten. Diese Ausbildung der Vorrichtung 1 ist grundsätzlich als gleichwertig zu der zuvor beschriebenen Ausbildung anzusehen. Die in den Fig. 1 und 2 dargestellte Ausbildung hat lediglich den Vorteil, dass durch den waagerechten im vertikal oberen Bereich der Flokkulationszone 5 einmündenden Zulauf 3 die Flokkulationszone 5 kürzer ausgebildet werden kann, da sich hier die Suspension zunächst vertikal nach unten und dann vertikal aufsteigend bewegt. Um gleiche Elektrodenflächen zu erhalten, muss demgegenüber bei der in der Fig. 3 wiedergegebenen Ausbildung die Flokkulationszone 5 verlängert (nahezu doppelte Länge) werden. Allerdings kommt diese Ausbildungsform dafür mit lediglich zwei Elektroden 8', 8" für das Elektrodensystem 8 der Flokkulationszone 5 aus. Wie ersichtlich, wird die Suspension der Vorrichtung 1 mittels einer Pumpe 19 aus einem Vorratsbehälter 2" des aus der Photosyntheseeinheit 2' und dem Behälter 2" bestehenden Bioreaktor 2 zugeführt. Auf diese Pumpe 19 kann jedoch verzichtet werden, wenn der Zulauf 3 zur Vorrichtung 1 über einen auf der Druckseite der anderen Pumpe 20 angeordneten Abzweig erfolgt, wobei die Durchflussmenge durch diesen Abzweig dann separat regelbar sein muss.

Das gesamte System aus Photobioreaktor 2 und (Ernte-)Vorrichtung wird durch Sensoren 16, 18 und eine Mess- und Steuereinrichtung 17 komplettiert. Mittels im Bereich des Zulaufs 3 angeordneter Sensoren 18 sind dabei beispielsweise eine pH-Wert-Messung oder eine Leitfähigkeitsmessung zur Ableitung von Stellgrößen für die Verfahrensführung in der Mess- und Steuereinheit 17 ermöglicht. Aus den erhaltenen Messwerten können Stellgrößen abgeleitet werden, mittels derer durch eine eventuelle CO₂-Dosierung im Bereich des Bioreaktors 2 oder durch eine Steuerung des zeitlichen Verlaufs der Ströme durch die Elektroden 8', 8", 8''', 9 der Verfahrensverlauf in einer für den Ernteerfolg bzw. die Effizienz günstigen Weise (s. Erläuterungen weiter oben) beeinflusst werden kann. Gemäß dem Beispiel nach der Fig. 3 ist außerdem ein Füllstandssensor 16 mit der Vorrichtung 1 gekoppelt, so dass über die Steuerung des Durchsatzes mittels in der Mess- und Steuereinrichtung 17 gewonnener Stellgrößen auf die Dicke der sich an der Oberfläche der Flotationszone 6 ausbildenden Schaumschicht 13 Einfluss genommen werden kann, um so eine möglichst trockene Biomasse zu ernten. Zu diesem Zweck kann beispielsweise die Leistung der Pumpe 19 oder der Durchfluss (hier nicht dargestellter) steuerbarer Ventile im Zulauf gesteuert werden.

### Aufstellung der verwendeten Bezugszeichen

- 1: (Ernte-)Vorrichtung
- 2: (Photo-)Bioreaktor
- 2': Photosyntheseeinheit
- 2": Sammel- und Ausgleichsbehälter
- 3: Zulauf
- 4: Ablauf
- 5: Flokkulationszone
- 6: Flotationszone
- 7: Anreicherungszone
- 8: Elektrodensystem
- 8': (Innen-)Elektrode
- 8": Rohrelektrode, (Außen-)Elektrode
- 8''': Rohrelektrode, (Zwischen-)Elektrode
- 9: (Gitter-)Elektroden, (Elektrodensystem)
- 10: (Grund-)platte
- 11: Scheibe
- 12: Unterseite (der Scheibe)
- 13: Schaum(-schicht)
- 14: Ablaufrinne
- 15: Kante (der Ablaufrinne)
- 16: Füllstandssensor
- 17: Mess- und Steuereinrichtung
- 18: Sensor (pH- oder Leitfähigkeitssensor)
- 19: Pumpe
- 20: Pumpe
- 21: Ablasshahn
- 22: Durchführung
- 23, 23': Stehbolzen
- 24: Isolator

## Patentansprüche

1. Verfahren zur Ernte von in einem offenen oder geschlossenen Kultivationssystem (2), vorzugsweise einem Bioreaktor, in wässriger Nährlösung kultivierter mikrobieller Biomasse, vorzugsweise von Mikroalgen, bei dem die Ernte der Biomasse unter Nutzung elektrokinetischer Mechanismen als kontinuierlicher Prozess in einer nach dem Durchflussprinzip arbeitenden Bypassanordnung zum Kultivationssystem (2) erfolgt, indem die Biomasse enthaltende Nährlösung (Suspension) als Volumenstrom durch ein als Flokkulationszone (5) wirkendes Elektrodensystem (8) geleitet und die hier unter Einwirkung einer an den Elektroden (8', 8", 8''') anliegenden Gleichspannung flokkulierende Biomasse in einer sich anschließenden Flotationszone (6) durch einen ebenfalls elektrolytisch mittels eines weiteren Elektrodensystems (9) erzeugten Gasbläschenstrom an die Oberfläche der Suspension getragen wird (Flotation), wo sich die Biomasse in Form eines Schaums (13) anreichert und schließlich abgeschöpft, abgenommen oder über eine Ablaufrinne (14) in einen Auffangbehälter abgeführt wird, während die aus der Suspension verbleibende Flüssigkeit in das Kultivationssystem (2) zurückgeführt wird, wobei die Suspension vertikal durch die Flokkulationszone (5) geführt wird und durch sich bei der Flokkulation bildendes Gas in einer Aufwärtsbewegung in die Flotationszone (6) einströmt, so dass die Flotation durch bereits bei der Flokkulation entstehende Gasbläschen eingeleitet und unterstützt wird.

2. Verfahren nach Anspruch 1, **dadurch gekennzeichnet, dass** bei der Bestromung der Elektroden (8', 8", 8''', 9) der Flokkulations- und der Flotationszone (5, 6) zyklisch die Polarität gewechselt wird.

3. Verfahren nach Anspruch 1 bis oder 2, **dadurch gekennzeichnet, dass** der Wassergehalt der geernteten Biomasse durch Variation des Füllstandes in der Flotationszone (6) beeinflussbar ist.

4. Verfahren nach einem der Ansprüche 1 bis 3, **dadurch gekennzeichnet, dass** im Zuge der Zuführung der Suspension zur Flokkulationszone (5) eine pH-Wert-Messung erfolgt und aus dem dabei festgestellten pH-Wert Stellgrößen für die Prozessführung im Hinblick auf eine Zudosierung von CO₂ und/oder eine Veränderung der Bestromung der Elektroden (8', 8", 8''') abgeleitet werden.

5. Verfahren nach einem der Ansprüche 1 bis 4, **dadurch gekennzeichnet, dass** auf der Grundlage einer Messung der optischen Dichte (Trübungsmessung) der in das Kultivationssystem (2) zurückgeführten Suspension Rückschlüsse auf die Konzentration von Biomasse und aus dem Verhältnis zu der im Kultivationssystem (2) vorhandenen Konzentration der Biomasse auf die Effizienz des Ernteverfahrens geschlossen wird.

6. Vorrichtung (1) zur Ernte von in einem offenen oder geschlossenen Kultivationssystem (2), vorzugsweise einem Bioreaktor, in wässriger Nährlösung kultivierter mikrobieller Biomasse, vorzugsweise von Mikroalgen, welche als Bypassanordnung mit einem Zulauf (3) vom und einem Ablauf (4) zum Kultivartionssystem (2) ausgebildet ist und in einem gemeinsamen Gehäuse eine Flokkulationszone (5), eine vertikal oberhalb der Flokkulationszone (5) angeordnete Flotationszone (6) sowie eine sich an die Flotationszone (6) in vertikaler Richtung anschließende Anreicherungszone (7) für die Biomasse umfasst, wobei es sich bei der Flokkulationszone (5) um einen mit dem Zulauf (3) unmittelbar in Verbindung stehenden und von der Biomasse enthaltenden Nährlösung (Suspension) durchflossenen Abschnitt mit einem Elektrodensystem (8) von senkrecht verlaufenden, horizontal benachbart angeordneten Elektroden (8', 8", 8''') handelt, welcher in einen sich in seiner horizontalen Erstreckung gegenüber der Flokkulationszone (5) erweiternden, die Flotationszone (6) ausbildenden Abschnitt mit einer Verbindung zum Ablauf (4) und mindestens einer waagerecht, vorzugsweise mehreren waagerecht und parallel zueinander darin eingeordneten Gitterelektroden (9) hineinragt, in dem sich beim Betrieb der Vorrichtung oberhalb der Gitterelektroden (9) und von ihnen beabstandet die Anreicherungszone (7) ausbildet.

7. Vorrichtung (1) nach Anspruch 6, **dadurch gekennzeichnet, dass** das Elektrodensystem (8) der Flokkulationszone (5) einerseits und die Gitterelektroden (9) der Flotationszone (6) andererseits von unabhängigen Stromquellen gespeist werden.

8. Vorrichtung (1) nach Anspruch 6 oder 7, **dadurch gekennzeichnet, dass** das Elektrodensystem (8) der Flokkulationszone (5) aus einem eine äußere Elektrode (8") ausbildenden metallischen Rohr und einer zweiten, konzentrisch darin eingeordneten inneren Elektrode (8') besteht, welche als metallischer Rundstab oder ein an beiden Enden verschlossenes Metallrohr ausgebildet ist, wobei die über den Zulauf (3) zugeführte Suspension zur Flokkulation der Biomasse zwischen der äußeren und der inneren Elektrode (8', 8") hindurchgeleitet wird.

9. Vorrichtung (1) nach Anspruch 6 oder 7, **dadurch gekennzeichnet, dass** das Elektrodensystem (8) der Flokkulationszone aus einer ersten, inneren als Rundstab oder beidseits verschlossenes Rohr ausgebildeten sowie konzentrisch in einer zweiten, äußeren Rohrelektrode (8") (Außenelektrode) angeordneten Elektrode (8') (Innenelektrode) sowie einer zwischen der Innen- und der Außenelektrode (8', 8") eingeordneten dritten Elektrode (8''') (Zwischenelektrode) gebildet ist, wobei die Suspension zur Flokkulation zuerst vertikal zwischen der Außen- und der Zwischenelektrode (8", 8''') sowie anschließend zwischen der Innen- und der Zwischenelektrode (8', 8") geführt wird und der Zulauf (3) waagerecht, kurz unterhalb der Flotationszone (6), in die Flokkulationszone (5) einmündet.

10. Vorrichtung (1) nach Anspruch 9, **dadurch gekennzeichnet, dass** der Zulauf (3) und der Ablauf (4) auf annähernd gleicher Höhe waagerecht in einer den Aufbau mit den Gitterelektroden (9) der Flotationszone (6) abstützenden Grundplatte (10) verlaufen.

11. Vorrichtung (1) nach Anspruch 8 oder 9, **dadurch gekennzeichnet, dass** das in die Flotationszone (6) hineinragende Ende des die Flokkulationszone (5) ausbildenden Abschnitts von einer sich radial nach außen erstreckenden in den Stromkreis der Gitterelektroden (9) einbezogenen Scheibe (11) eingefasst ist, bei welcher zumindest die die Unterseite (12) ausbildende große Außenfläche gegen die Horizontale geneigt verläuft, so dass sich die Dicke der Scheibe (11) von ihrem Zentrum nach außen verringert.

12. Vorrichtung (1) nach einem der Ansprüche 6 bis 11, **dadurch gekennzeichnet, dass** am oberen Abschluss der Flotationszone (6), im Bereich der sich beim Betrieb der Vorrichtung (1) ausbildenden Anreicherungszone (7), eine nach unten geneigt verlaufende Ablaufrinne (14) angeordnet ist, über welche die Biomasse unter Ausnutzung der Schwerkraft einem Auffangbehälter zugeführt wird.

13. Vorrichtung (1) nach Anspruch 12, **dadurch gekennzeichnet, dass** Mittel (16, 17) zur Regulierung des Füllstands in der Flotationszone (6) vorgesehen sind, so dass sich die Anreicherungszone (7) in Form des mit der Biomasse angereicherten Schaums (13) beim Betrieb der Vorrichtung (1) etwa von 2 bis 5 cm unterhalb der Kante (15) zur Ablaufrinne (14) vertikal nach oben erstreckt.

14. Vorrichtung (1) nach Anspruch 12 oder 13, **dadurch gekennzeichnet, dass** auf dem Boden der Ablaufrinne (14), bezogen auf die Bewegungsrichtung des aus der Anreicherungszone (7) in den Auffangbehälter ausgetragenen bzw. ablaufenden Schaums, Querrinnen ausgebildet sind.

15. Vorrichtung (1) nach einem der Ansprüche 6 bis 14, **dadurch gekennzeichnet, dass** diese über Sensoren (16, 18) zur pH- und/oder Leitfähigkeitsmessung sowie zur Messung des Füllstandes in der Flotationszone und eine mit diesen Sensoren verbundene Mess- und Steuervorrichtung (17) verfügt.

16. Vorrichtung (1) nach Anspruch 15, **dadurch gekennzeichnet, dass** durch den Füllstandssensor (16) und die mit ihm verbundene Mess- und Steuervorrichtung (17) ein Regelungskreis für eine Füllstandsregelung gebildet ist.

17. Vorrichtung (1) nach einem der Ansprüche 6 bis 16, **dadurch gekennzeichnet, dass** die Zuführung der Suspension über den Zulauf (3) zur Flokkulationszone (5) mittels einer zwischen einem Sammel- und Ausgleichsbehälter (2") eines als Bioreaktor ausgebildeten Kultivationssystems (2) und der Vorrichtung (1) angeordneten Pumpe (19) erfolgt.

18. Vorrichtung (1) nach einem der Ansprüche 6 bis 16, **dadurch gekennzeichnet, dass** die Zuführung der Suspension zur Flokkulationszone (5) über einen Abzweig erfolgt, welcher auf der Druckseite einer zwischen einer Photosyntheseeinheit (2') und einem Sammel- und Ausgleichsbehälter (2") eines als Photobioreaktor ausgebildeten Kultivationssystems (2) vorhandenen Pumpe (20) angeordnet ist, wobei in dem Abzweig Mittel zur gesonderten Einstellung des Volumenstroms zur Flokkulationszone (5) angeordnet sind.

## Claims

1. Process for the harvesting of microbial biomass, preferably of microalgae, cultivated in aqueous nutrient solution in an open or closed cultivation system (2), preferably a bioreactor, in which the harvesting of the biomass takes place using electrokinetic mechanisms as a continuous process in a bypass arrangement, working on the throughflow principle, to the cultivation system (2), in which the biomass-containing nutrient solution (suspension) is passed as a volume flow through an electrode system (8) acting as a flocculation zone (5) and the biomass flocculating here under the action of a DC voltage applied to the electrodes (8', 8", 8"') is taken in a following flotation zone (6) by a gas bubble flow likewise produced electrolytically by means of a further electrode system (9) to the surface of the suspension (flotation) where the biomass accumulates in the form of a foam (13) and is ultimately skimmed off, removed or led off via a drainage channel (14) into a receiving tank, whilst the liquid remaining from the suspension is returned into the cultivation system (2), wherein the suspension is led vertically through the flocculation zone (5) and flows through gas forming during flocculation in an upwards movement into the flotation zone (6), so that the flotation is introduced and supported by gas bubbles produced during flocculation.

2. Process according to claim 1, **characterised in that** when the electrodes (8', 8", 8"', 9) flow through the flocculation and flotation zones (5, 6) the polarity is changed cyclically.

3. Process according to claim 1 or 2, **characterised in that** the water content of the harvested biomass can be influenced by variation of the fill level in the flotation zone (6).

4. Process according to one of claims 1 to 3, **characterised in that** a pH measurement takes place in the course of feeding of the suspension to the flocculation zone (5) and from the pH determined thereby, control variables are derived for carrying out the process with regard to a feed of CO₂ and/or a change to the flow of the electrodes (8', 8", 8"').

5. Process according to one of claims 1 to 4, **characterised in that** on the basis of a measurement of the optical density (turbidity measurement) of the suspension returned into the cultivation system (2), conclusions are drawn on the concentration of biomass and from the relation to the concentration of the biomass present in the cultivation system (2) on the efficiency of the harvesting process.

6. Device (1) for the harvesting of microbial biomass, preferably of microalgae, cultivated in aqueous nutrient solution in an open or closed cultivation system (2), preferably a bioreactor, which is formed as a bypass arrangement with an inflow (3) from and an outflow (4) to the cultivation system (2) and comprises in a common housing a flocculation zone (5), a flotation zone (6) arranged vertically above the flocculation zone (5) and an accumulation zone (7) connecting in a vertical direction to the flotation zone (6) for the biomass, wherein the flocculation zone (5) is a section connected directly to the inflow (3) and through which flows the biomass-containing nutrient solution (suspension) with an electrode system (8) of electrodes (8', 8", 8"') running vertically arranged horizontally adjacent, which projects into a section expanding in its horizontal extension with regard to the flocculation zone (5) forming the flotation zone (6) with a connection to the outflow (4) and at least one grid electrode (9) fitted therein horizontally, preferably several fitted therein horizontally and parallel to one another, in which on operation of the device the accumulation zone (7) forms above the grid electrodes (9) and spaced from them.

7. Device (1) according to claim 6, **characterised in that** the electrode system (8) of the flocculation zone (5) on the one hand and the grid electrodes (9) of the flotation zone (6) on the other hand is fed by independent power sources.

8. Device (1) according to claim 6 or 7, **characterised in that** the electrode system (8) of the flocculation zone (5) consists of a metal pipe forming an outer electrode (8") and a second inner electrode (8') fitted concentrically therein, which is formed as a metal round rod or a metal pipe closed at both ends, wherein the suspension fed via the inflow (3) is passed to the flocculation of the biomass between the outer and the inner electrodes (8', 8").

9. Device (1) according to claim 6 or 7, **characterised in that** the electrode system (8) of the flocculation zone is formed from a first inner electrode (8') (inner electrode) arranged as a round rod or pipe closed on both sides and concentrically in a second, outer pipe electrode (8") (outer electrode), and a third electrode (8"') fitted between the inner and outer electrodes (8', 8") (intermediate electrode), wherein the suspension is led to the flocculation first of all vertically between the outer and the intermediate electrodes (8", 8'") and then between the inner and intermediate electrodes (8', 8"), and the inflow (3) leads horizontally into the flocculation zone (5) just below the flotation zone (6).

10. Device (1) according to claim 9, **characterised in that** the inflow (3) and outflow (4) run at approximately the same height horizontally in a base plate (10) supporting the assembly with the grid electrodes (9) of the flotation zone (6).

11. Device (1) according to claim 8 or 9, **characterised in that** the end, projecting into the flotation zone (6), of the section forming the flocculation zone (5) is bordered by a disk (11) extending radially outwards into the electric circuit of the grid electrodes (9), in which at least the large external surface forming the underside (12) runs at an angle to the horizontal, so that the thickness of the disk (11) is reduced outwards from its centre.

12. Device (1) according to one of claims 6 to 11, **characterised in that** at the upper end of the flotation zone (6), in the area of the accumulation zone (7) forming on operation of the device (1), is arranged a drainage channel (14) running downwards at an angle, via which the biomass is fed using gravity to a receiving tank.

13. Device (1) according to claim 12, **characterised in that** means (16, 17) for regulating the fill level in the flotation zone (6) are provided so that the accumulation zone (7) in the form of the foam (13) accumulated with the biomass extends vertically upwards to the discharge channel (14) on operation of the device (1) approximately 2 to 5 cm below the edge (15).

14. Device (1) according to claim 12 or 13, **characterised in that** transverse channels are formed on the bottom of the drainage channel (14), based on the direction of movement of the foam discharged or draining from the accumulation zone (7) into the receiving tank.

15. Device (1) according to one of claims 6 to 14, **characterised in that** this has sensors (16, 18) to measure the pH and/or conductivity and to measure the fill level in the flotation zone and a measuring and control device (17) connected to these sensors.

16. Device (1) according to claim 15, **characterised in that** a regulating circuit for fill level regulation is formed by the fill level sensor (16) and the measuring and control device (17) connected to it.

17. Device (1) according to one of claims 6 to 16, **characterised in that** feeding of the suspension takes place via the inflow (3) to the flocculation zone (5) by means of a pump (19) arranged between a collecting and compensating tank (2") of a cultivation system (2) formed as a bioreactor and the device (1).

18. Device (1) according to one of claims 6 to 16, **characterised in that** feeding of the suspension to the flocculation zone (5) takes place via a branch which is arranged on the pressure side of a pump present between a photosynthesis unit (2') and a collecting and compensating tank (2") of a cultivation system (2) formed as a photobioreactor, wherein means for the separate setting of the volume flow to the flocculation zone (5) are arranged in the branch.

## Revendications

1. Procédé de récolte de biomasse microbienne, de préférence des microalgues, cultivée en solution nutritive aqueuse dans un système de culture ouvert ou fermé (2), de préférence un bioréacteur, dans lequel la récolte de la biomasse s'effectue en utilisant des mécanismes électrocinétiques en processus continu dans un agencement de dérivation fonctionnant selon le principe de circulation vers le système de culture (2), en guidant la solution nutritive contenant la biomasse (suspension) sous forme de courant volumique à travers un système d'électrodes (8) agissant en tant que zone de floculation (5) et en amenant la biomasse floculante sous l'effet d'une tension continue appliquée aux électrodes (8', 8", 8"') dans une zone de flottation suivante (6) par un courant de bulles d'air produit également de manière électrolytique au moyen d'un autre système d'électrodes (9), contre la surface de la suspension (flottation), où la biomasse se concentre sous forme de mousse (13) et finalement est écumée, est enlevée ou est évacuée par le biais d'une rigole d'évacuation (14) dans un récipient collecteur, tandis que le liquide subsistant de la suspension est ramené dans le système de culture (2), la suspension étant guidée verticalement à travers la zone de floculation (5) et affluant dans la zone de flottation (6) suivant un mouvement ascendant sous l'effet du gaz se formant au cours de la floculation, de sorte que la flottation soit amorcée et favorisée par les bulles de gaz se produisant déjà au cours de la floculation.

2. Procédé selon la revendication 1, **caractérisé en ce que** lors de l'alimentation électrique des électrodes (8', 8", 8"', 9) de la zone de floculation et de flottation (5, 6), la polarité est inversée cycliquement.

3. Procédé selon la revendication 1 ou 2, **caractérisé en ce que** la teneur en eau de la biomasse récoltée peut être influencée en faisant varier le niveau dans la zone de flottation (6).

4. Procédé selon l'une quelconque des revendications 1 à 3, **caractérisé en ce qu'**au cours de l'alimentation de la suspension vers la zone de floculation (5), une mesure du pH est effectuée et à partir de la valeur de pH ainsi établie, on déduit des valeurs de réglage pour la conduite d'un processus en termes de dosage de CO₂ et/ou de modification de l'alimentation électrique des électrodes (8', 8", 8"').

5. Procédé selon l'une quelconque des revendications 1 à 4, **caractérisé en ce que** sur la base d'une mesure de la densité optique (mesure de la turbidité) de la suspension recirculée dans le système de culture (2), on peut en déduire la concentration de la biomasse et à partir du rapport à la concentration existante de la biomasse existante dans le système de culture (2), on peut en déduire le rendement du procédé de récolte.

6. Dispositif (1) de récolte de biomasse microbienne, de préférence des microalgues, cultivée en solution nutritive aqueuse dans un système de culture ouvert ou fermé (2), de préférence un bioréacteur, qui est réalisé sous forme d'agencement de dérivation avec une entrée (3) depuis et une sortie (4) vers le système de culture (2), et qui comprend dans un boîtier commun une zone de floculation (5), une zone de flottation (6) disposée verticalement au-dessus de la zone de floculation (5) ainsi qu'une zone de concentration (7) pour la biomasse se raccordant à la zone de flottation (6) dans la direction verticale, la zone de floculation (5) étant une portion connectée directement à l'entrée (3) et parcourue par la solution nutritive (suspension) contenant la biomasse, avec un système d'électrodes (8) constitué d'électrodes (8', 8", 8"') s'étendant verticalement, disposées de manière adjacente horizontalement, cette portion entrant dans une portion s'élargissant dans son étendue horizontale par rapport à la zone de floculation (5), constituant la zone de flottation (6), avec une connexion à la sortie (4) et au moins une électrode de grille (9) disposées horizontalement, de préférence plusieurs électrodes de grille (9) disposées horizontalement et parallèlement les unes aux autres dans celle-ci, dans laquelle se forme la zone de concentration (7) lors du fonctionnement du dispositif, au-dessus des électrodes de grille (9) et à distance de celles-ci.

7. Dispositif (1) selon la revendication 6, **caractérisé en ce que** le système d'électrodes (8) de la zone de floculation (5) d'une part et les électrodes de grille (9) de la zone de flottation (6) d'autre part sont alimentées par des sources de courant indépendantes.

8. Dispositif (1) selon la revendication 6 ou 7, **caractérisé en ce que** le système d'électrodes (8) de la zone de floculation (5) se compose d'un tube métallique constituant une électrode extérieure (8") et d'une deuxième électrode intérieure (8') disposée concentriquement dans celui-ci, qui est réalisée sous la forme d'une barre ronde métallique ou d'un tube métallique fermé aux deux extrémités, la suspension alimentée par le biais de l'entrée (3) étant guidée en vue de la floculation de la biomasse entre les électrodes extérieure et intérieure (8', 8").

9. Dispositif (1) selon la revendication 6 ou 7, **caractérisé en ce que** le système d'électrodes (8) de la zone de floculation se compose d'une première électrode intérieure (8') (électrode intérieure) réalisée en tant que barre ronde ou de tube fermé des deux côtés et disposée concentriquement dans une deuxième électrode tubulaire extérieure (8") (électrode extérieure), ainsi que d'une troisième électrode (8"') (électrode intermédiaire) disposée entre l'électrode intérieure et l'électrode extérieure (8', 8"), la suspension étant guidée en vue de la floculation d'abord verticalement entre l'électrode extérieure et l'électrode intermédiaire (8", 8"') et ensuite entre l'électrode intérieure et l'électrode intermédiaire (8', 8"'), et l'entrée (3) débouchant horizontalement, juste en dessous de la zone de flottation (6), dans la zone de floculation (5).

10. Dispositif (1) selon la revendication 9, **caractérisé en ce que** l'entrée (3) et la sortie (4) s'étendent approximativement à la même hauteur horizontalement dans une plaque de base (10) supportant la structure avec les électrodes de grille (9) de la zone de flottation (6).

11. Dispositif (1) selon la revendication 8 ou 9, **caractérisé en ce que** l'extrémité entrant dans la zone de flottation (6) de la portion constituant la zone de floculation (5) est encadrée par un disque (11) s'étendant radialement vers l'extérieur et inclus dans le circuit électrique des électrodes de grille (9), dans lequel disque au moins la grande surface extérieure constituant le côté inférieur (12) est inclinée par rapport à l'horizontale, de sorte que l'épaisseur du disque (11) diminue depuis son centre vers l'extérieur.

12. Dispositif (1) selon l'une quelconque des revendications 6 à 11, **caractérisé en ce qu'**au niveau de la terminaison supérieure de la zone de flottation (6), dans la région de la zone de concentration (7) se formant lors du fonctionnement du dispositif (1), est disposée une rigole d'évacuation (14) s'étendant de manière inclinée vers le bas, par le biais de laquelle la biomasse est acheminée à un récipient de collecte en utilisant la force de gravité.

13. Dispositif (1) selon la revendication 12, **caractérisé en ce que** des moyens (16, 17) sont prévus pour réguler le niveau de remplissage dans la zone de flottation (6), de sorte que la zone de concentration (7) s'étende verticalement vers le haut sous forme de mousse (13) concentrée avec la biomasse lors du fonctionnement du dispositif (1), à environ 2 cm à 5 cm en dessous de l'arête (15) de la rigole d'évacuation (14).

14. Dispositif (1) selon la revendication 12 ou 13, **caractérisé en ce qu'**au fond de la rigole d'évacuation (14), par rapport au sens de déplacement de la mousse déchargée ou s'écoulant de la zone de concentration (7) dans le récipient collecteur, sont réalisées des rigoles transversales.

15. Dispositif (1) selon l'une quelconque des revendications 6 à 14, **caractérisé en ce que** celui-ci dispose de capteurs (16, 18) pour mesurer le pH et/ou la conductibilité ainsi que pour mesurer le niveau de remplissage dans la zone de flottation, et d'un dispositif de mesure et de commande (17) associé à ces capteurs.

16. Dispositif (1) selon la revendication 15, **caractérisé en ce que** le capteur de niveau de remplissage (16) et le dispositif de mesure et de commande (17) associé permettent de former un circuit de régulation pour une régulation du niveau.

17. Dispositif (1) selon l'une quelconque des revendications 6 à 16, **caractérisé en ce que** l'alimentation de la suspension s'effectue par le biais de l'entrée (3) vers la zone de floculation (5) au moyen d'une pompe (19) disposée entre un récipient collecteur et de compensation (2") d'un système de culture (2) réalisé en tant que bioréacteur, et le dispositif (1).

18. Dispositif (1) selon l'une quelconque des revendications 6 à 16, **caractérisé en ce que** l'alimentation de la suspension vers la zone de floculation (5) s'effectue par le biais d'une dérivation qui est disposée du côté pression d'une pompe (20) prévue entre une unité de photosynthèse (2') et un récipient collecteur et de compensation (2") d'un système de culture (2) réalisé sous la forme d'un photobioréacteur, des moyens pour l'ajustage séparé du courant volumique vers la zone de floculation (5) étant disposés dans la dérivation.
